# EUROPEAN PATENT APPLICATION

(11) **EP 3 196 207 A1**
(43) Date of publication of application: **26.07.2017**
(21) Application number: 16153974.7
(22) Date of filing: 02.02.2016
(51) Int. Cl.: C07K 1/02, C07K 1/04

(54) **METHOD FOR PREPARATION OF PEPTIDES WITH PSWANG LINKER**

(30) Priority: 20.01.2016 EP 16152145
(71) Applicant: Lonza Ltd, 3930 Visp (CH)
(72) Inventor: The designation of the inventor has not yet been filed

(57) **Abstract**

The invention discloses a method for the preparation of a peptide by liquid phase coupling of two fragments, an N-terminal fragment and a C-terminal fragment of the desired peptide, wherein the C-terminal fragment is protected on its C-terminal COOH by a psWang linker; the method is demonstrated with liraglutide wherein the C-terminal fragment carries the Palmitoyl-Glu-OtBu residue on the Lys⁽²⁰⁾.

SEQUENCE LISTING

<110>  Lonza Braine S.A.

<120>  Method for Preparation of Peptides with psWang Linker

<130>  LP2433EP01

<150>  EP16152145.5
<151>  2016-01-20

<160>  3

<170>  PatentIn version 3.5

<210>  1
<211>  16
<212>  PRT
<213>  Artificial

<220>
<223>  Synthetic construct

<400>  1

His Ala Glu Gly Thr Phe Thr Ser Asp Val Ser Ser Tyr Leu Glu Gly
1               5                   10                  15

<210>  2
<211>  15
<212>  PRT
<213>  Artificial

<220>
<223>  Synthetic construct

<400>  2

Gln Ala Ala Lys Glu Phe Ile Ala Trp Leu Val Arg Gly Arg Gly
1               5                   10                  15

<210>  3
<211>  31
<212>  PRT
<213>  Artificial

<220>
<223>  Synthetic construct

<400>  3

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Arg Gly Arg Gly
            20                  25                  30

SEQUENCE LISTING

<110>  Lonza Braine S.A.

<120>  Method for Preparation of Peptides with psWang Linker

<130>  LP2433EP01

<150>  EP16152145.5
<151>  2016-01-20

<160>  3

<170>  PatentIn version 3.5

<210>  1
<211>  16
<212>  PRT
<213>  Artificial

<220>
<223>  Synthetic construct

<400>  1

His Ala Glu Gly Thr Phe Thr Ser Asp Val Ser Ser Tyr Leu Glu Gly
1               5                   10                  15


<210>  2
<211>  15
<212>  PRT
<213>  Artificial

<220>
<223>  Synthetic construct

<400>  2

Gln Ala Ala Lys Glu Phe Ile Ala Trp Leu Val Arg Gly Arg Gly
1               5                   10                  15


<210>  3
<211>  31
<212>  PRT
<213>  Artificial

<220>
<223>  Synthetic construct

<400>  3

His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Arg Gly Arg Gly
            20                  25                  30

## Description

The invention discloses a method for the preparation of a peptide by liquid phase coupling of two fragments, an N-terminal fragment and a C-terminal fragment of the desired peptide, wherein the C-terminal fragment is protected on its C-terminal COOH by a psWang linker; the method is demonstrated with liraglutide wherein the C-terminal fragment carries the Palmitoyl-Glu-OtBu residue on the Lys⁽²⁰⁾.

### BACKGROUND OF THE INVENTION

In convergent peptide synthesis, two fragments, the N-terminal fragment and the C-terminal fragment, of a desired peptide are synthesized by SPPS, thereafter these two fragments are coupled by LPPS. The disadvantage of this method is that the C-terminal COOH of the C-terminal fragment is obtained after cleavage from the resin after SPPS in its unprotected form. Therefore it first must be protected again before this C-terminal fragment can be used in the liquid phase coupling with the N-terminal fragment.
This reprotection of the C-terminal COOH of the C-terminal fragment after SPPS is an additional step with additional isolation etc.

There was a need for a method for preparation of peptides with convergent peptide synthesis that does not require this reprotection of the C-terminal COOH of the C-terminal fragment after SPPS.

By the use of a psWang handle during the preparation by SPPS of the C-terminal fragment it is possible to isolate the C-terminal fragment already with the C-terminal COOH in a protected form after cleavage from the resin, thereby the described reprotection is no longer necessary. The method can be applied to any peptide.

The application of this method is illustrated with liraglutide.
Liraglutide has the CAS number 204656-20-2 and is compound of formula (3), 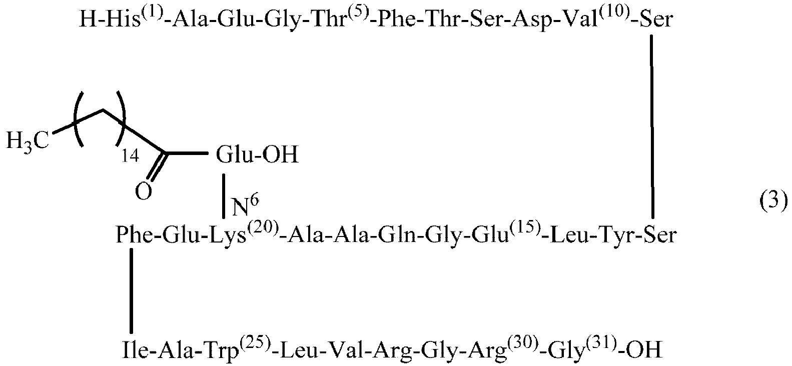 the sequence can also be described as follows:
H-His⁽¹⁾-Ala-Glu-Gly-Thr⁽⁵⁾-Phe-Thr-Ser-Asp-Val⁽¹⁰⁾-Ser-Ser-Tyr-Leu-Glu⁽¹⁵⁾-Gly-Gln-Ala-Ala-Lys⁽²⁰⁾⁽Palmitoyl-Glu)-Glu-Phe-Ile-Ala-Trp⁽²⁵⁾-Leu-Val-Arg-Gly-Arg⁽³⁰⁾-Gly⁽³¹⁾-OH;
the numbers in parenthesis show the numbering of the positions of the AA in the sequence as the numbering is used in instant invention.

WO 98/08871 A1 discloses a method for preparation of derivatives of GLP-1 and analogues thereof by culturing a host cell culture containing a DNA sequence encoding the polypeptide and capable of expressing the polypeptide in a suitable nutrient medium under conditions permitting the expression of the peptide, after which the resulting peptide is recovered from the culture.

WO 00/55119 A1 discloses the acylation of the epsilon-amino residue of Lys⁽²⁰⁾ of GLP-1 analogues with certain compounds useful as acylating agents. In this disclosure the N-terminal amino residue of GLP-1 analogues are not protected. The disadvantage is that diacylation occurs, both the N-terminal amino residue and the epsilon-amino residue of Lys⁽²⁰⁾ are acylated. The undesired by products need to be separated by purification.

In case of liraglutide, another advantage of the method is that the Palmitoyl-Glu-OtBu residue on the Lys⁽²⁰⁾ of N-terminal fragment can be introduced onto the N-terminal fragment during SPPS and on-resin, while the N-terminal NH₂ is still protected. If it was introduced onto a fully deprotected liraglutide precursor that has not yet the Palmitoyl-Glu-OtBu residue, a method that could be derived from the disclosure of WO 00/55119 A1, then the N-terminal NH₂ first needs to be selectively protected before the Palmitoyl-Glu-OtBu residue can be introduced onto the Lys⁽²⁰⁾ in order to avoid diacylation. This required selective protection is additional difficulty, an additional source of impurities and introduces further steps in the synthesis of liraglutide.
In case of liraglutide, another advantage of the method is that the Palmitoyl-Glu-OtBu residue can be introduced with a good yield and with low side reactions by introducing it after the coupling of Ala⁽¹⁸⁾ or after the coupling of Ala⁽¹⁹⁾, thereby no or only low amounts of by products are formed, which would need to be separated.

In the text, the following meanings are used:
- AA: amino acid or amino acid residue, depending on context;
- Acm: acetamidomethyl;
- Alloc: allyloxycarbonyl protecting group;
- Boc: tert-butoxycarbonyl protecting group;
- coupling: also called coupling reaction;
- CTC: chlorotritylchloride;
- DCM: dichloromethane;
- DIC: Diisopropylcarbodiimide;
- DIPEA: N,N-diisopropylethylamine;
- DMAP: N,N-4-Dimethylaminopyridine;
- DMF: Dimethylformamide;
- DMSO: Dimethylsulfoxide;
- Dnp: dinitrophenyl;
- eq: equivalents
- Fmoc: 9-fluorenylmethoxycarbonyl protecting group;
- HAL: 5-(4-hydroxymethyl-3,5-dimethoxyphenoxy)valeric acid;
- HFIP: hexafluoroisopropanol;
- HMPB: 4-(4-hydroxymethyl-3-methoxyphenoxy)butyric acid;
- MIS: 1,2-dimethylindole-3-sulfonyl;
- N⁶: denotes the amino residue of the side chain of Lys;
- LPPS: Liquid Phase Peptide Synthesis;
- Mtr: 4-methoxy-2,3,6-trimethylphenylsulfonyl;
- N-terminal: NH₂ N-terminal amino residue;
- OxymaPure®: Ethyl (hydroxyimino)cyanoacetate, CAS 3849-21-6, purchased from Luxembourg Bio Technologies.

Pal, Palm abbreviations for Palmitoyl;
- Pbf: 2,2,4,6,7-pentamethyldihydrobenzofuran-5-sulfonyl protecting group;
- Pmc: 2,5,7,8-pentamethylchroman-6-sufonyl;
- psWang: pseudo Wang linker, also called pseudo Wang handle, in case of covalent connection to a resin or to a peptide it is also called a psWang residue;
- PyBOP: benzotriazol-1-yl-oxytripyrrolidinophosphonium hexafluorophosphate; 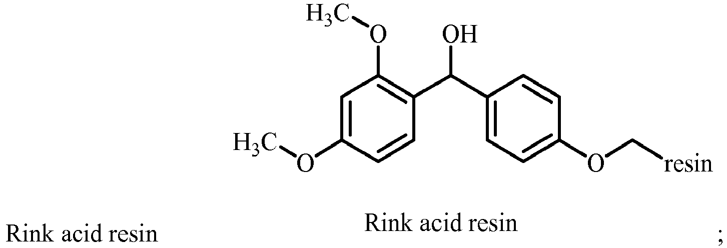
- RT: room or ambient temperature; 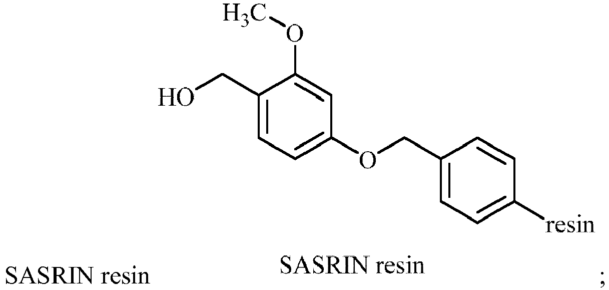
- SPPS: Solid Phase Peptide Synthesis;
- tBu: tert butyl or tBu protecting group;
- OtBu: tert butyl ester;
- TFA: trifluoroacetic acid;
- TIS: triisopropylsilane;
- Trt: trityl protecting group;
- Z: benzyloxycarbonyl;
if not otherwise stated.

### SUMMARY OF THE INVENTION

Subject of the invention is a method for the preparation of a peptide PEP which comprises a coupling of two peptide fragments, an N-terminal fragment FRAG1 of PEP and a C-terminal fragment FRAG2 of PEP, by liquid phase peptide synthesis;
wherein the C-terminal COOH of FRAG2 is protected by a psWang residue;
the psWang residue is residue of formula (psWang); 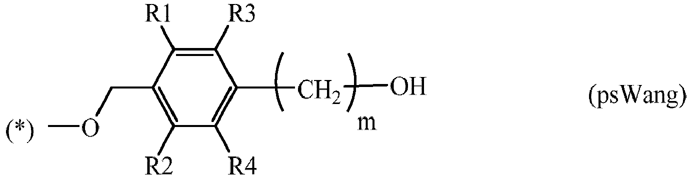
wherein the (*) denotes the covalent bond to the C atom of the CO residue of the C-terminal COOH of FRAG2;
R1, R2, R3 and R4 are identical or different and independently from each other selected from the group consisting of H, C₁₋₄ alkyl, C₁₋₄ alkoxy, CN, F, Cl and Br;
m is 0, 1, 2, 3, 4, 5, 6, 7, 8, 9 or 10.

### DETAILED DESCRIPTION OF THE INVENTION

Preferably, R1, R2, R3 and R4 are identical or different and independently from each other selected from the group consisting of H, C₁₋₂ alkyl, C₁₋₂ alkoxy and Cl;
more preferably, R1, R2, R3 and R4 are identical or different and independently from each other selected from the group consisting of H, methyl, methoxy and Cl;
even more preferably, R1, R2, R3 and R4 are H;
preferably at least two of the substituents R1, R2, R3 and R4 being H;
more preferably at least three of the substituents R1, R2, R3 and R4 being H.

Preferably, m is 0, 1, 2, 3 or 4;
more preferably, m is 0, 1 or 2;
more preferably, m is 0.

Especially, m is 0 and R1, R2, R3 and R4 are H, thereby the psWang residue is the p-hydroxymethylphenol residue, the residue of formula (psWang-1); 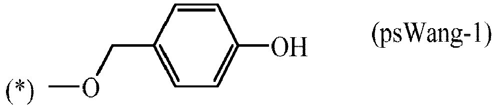 wherein the (*) denotes the covalent bond to the C atom of the CO residue of C-terminal COOH of FRAG 2.

The psWang residue is derived from the unconnected psWang linker, which is compound of formula (psWangLINK); 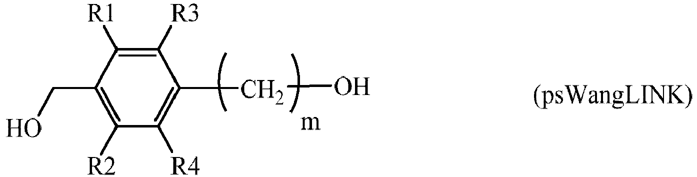 with m, R1, R2, R3 and R4 as defined herein, also with all their embodiments.

Preferably, FRAG2 is prepared by SPPS.

More preferably, FRAG2 is prepared by SPPS wherein the C-terminal amino acid residue is covalently connected to the psWang residue, and the psWang residue is covalently connected to the resin used for the SPPS.

Even more preferably, FRAG2 is prepared by SPPS wherein the alpha COOH of the C-terminal amino acid residue is covalently connected to the psWang residue, and the psWang residue is covalently connected to the resin used for the SPPS.

Preferably, FRAG2 is prepared by SPPS on a resin used for the SPPS loaded with the psWang linker.

Preferably, the psWang linker or the psWang residue respectively is connected to the resin used for the SPPS by an ester bond ESTBOND2;
so the resin used for the SPPS of FRAG2 is a resin that allows to connect the psWang linker or the psWang residue respectively to the resin by ESTBOND2.

Especially, FRAG2 is prepared by SPPS on a resin loaded with the psWang linker by firstly covalently bonding the psWang linker onto the resin by ESTBOND2, this is done by an esterification reaction ESTERREAC forming ESTBOND2. Thereafter preferably the C-terminal AA of FRAG2 is covalently bonded to the psWang linker that is on the resin, this is done by an esterification reaction, thereafter the other AA are coupled by SPPS onto the C-terminal AA and then onto the growing peptide chain respectively.

Preferably, ESTBOND2 is cleavable under weakly acidic conditions, with the weakly acidic conditions as described herein;
so the resin used for the SPPS of FRAG2 is a resin, that allows ESTBOND2 to be cleaved under weakly acidic conditions.

Preferably, ESTERREAC is done in the presence of a base. Suitable bases are for example trialkylamines, like N,N-diisopropylethylamine (DIPEA) or triethylamine (TEA); N,N-dialkylanilines, like N,N-diethylaniline; 2,4,6-trialkylpyridines, like 2,4,6-trimethylpyridine; and N-alkylmorpholines, like N-methylmorpholine.

Preferably, ESTERREAC is done in the presence of DIPEA as a base.

Preferably, the molar amount of base is from 1 to 10 times of the molar loading capacity of the resin.

Preferably, ESTERREAC can be done in any inert solvent which can dissolve the reactants. Preferred solvents for the coupling are water-miscible solvents like dimethyl sulfoxide (DMSO), dioxane, tetrahydrofurans such as tetrahydrofurane (THF) or methyltetrahydrofurane (Methyl-THF), 1-methyl-2-pyrrolidone (NMP), N,N-Dimethylformamide (DMF), N,N-Dimethylacetamide (DMA), or any mixture thereof; and non water-miscible solvents like dichloromethane (DCM), ethyl acetate or any mixture thereof; and any suitable mixture between water-miscible and non water-miscible solvents, including mixtures with water.

More preferably, ESTERREAC is done in a solvent selected from the group consisting of DMSO, dichloromethane, Me-THF, DMF, NMP, and mixtures thereof.

Preferably, the ESTERREAC is done at atmospheric pressure.

Preferably, the reaction temperature of the ESTERREAC is from -20 to 70°C; more preferably from -5 and 40°C, even more preferably from 0 to 35°C.

Preferably, the reaction time of the ESTERREAC is from 30 min to 12 h, more preferably from 30 min to 6 h, even more preferably from 30 min to 4 h.

The covalent bonding during SPPS of the alpha COOH of the C-terminal AA of FRAG2 to the psWang, and the covalent bonding of the psWang residue to the resin, are shown in formula (FRAG2-psWang-RESIN). 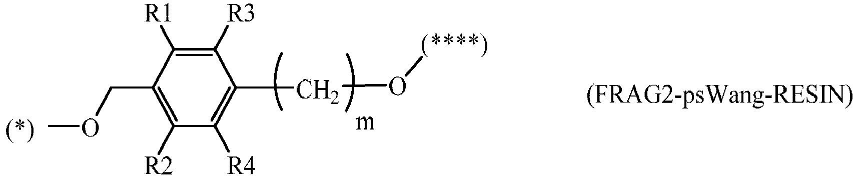 wherein
the (*) denotes the covalent bond to the C atom of the CO residue of the alpha COOH of the C-terminal AA of FRAG2;
the (****) denotes the covalent bond to the resin, preferably ESTBOND2.

Preferably, the method comprises two steps, a step STEP1 and a step STEP2;
STEP1 comprises the coupling of FRAG1 and FRAG2 by liquid phase peptide synthesis; STEP1 provides a peptide PEP-psWang;
STEP2 comprises cleavage of psWang from PEP-psWang obtained in STEP1.

Preferably, STEP2 provides PEP.

Preferably, the N-terminal NH₂ of FRAG1 is protected by a protecting group PROTGN during the coupling of FRAG1 and FRAG2.

PROTGN can be any protecting group suitable for protecting the N-terminal NH₂ of FRAG1. Commonly used PROTGN include protecting groups such as Boc, Fmoc, acetyl, Z or Trt.

More preferably, PROTGN is Boc.

Preferably, STEP1 provides the peptide PROTGN-PEP-psWang.

Preferably,
FRAG1 is 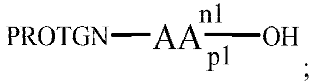 and
FRAG2 is 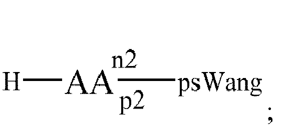
   p1 is an integer from 2 to 50 and denotes the total number of amino acid residues of FRAG1;
   p2 is an integer from 2 to 50 and denotes the total number of amino acid residues of FRAG2;
   n1 is an integer from 1 to p1 and denotes the amino acid residue in position n1 of FRAG 1;
   n2 is an integer from 1 to p2 and denotes the amino acid residue in position n2 of FRAG2;
   AA⁽ⁿ¹⁾ and AA⁽ⁿ²⁾ are the amino acid residues of FRAG1 and of FRAG2 on position n1 and on position n2 respectively, and are identical or different and independently from each other alpha amino acid residues.

Preferably,
p1 is an integer from 2 to 30;
p2 is an integer from 2 to 30;
more preferably,
p1 is an integer from 2 to 20;
p2 is an integer from 2 to 20.

Preferably, the amino acid residue of FRAG1 and FRAG2 are alpha amino acids residues which occur in natural peptides or in natural proteins.

More preferably, the amino acid residue of FRAG1 and FRAG2 are selected from the group consisting of Ala, Arg, Asn, Asp, Cys, Glu, Gln, Gly, His, Ile, Leu, Lys, Met, Phe, Pro, Ser, Thr, Trp, Tyr and Val.

Preferably, any amino acid residue of FRAG1 and FRAG2, that can be used in a side chain protected form, is used in side chain protected form.

In case of side chain protected amino acid residue of FRAG1 and FRAG2, STEP1 provides side chain protected PEP-psWang.

Amino acid residues of FRAG1 and FRAG2, that can be used in a side chain protected form and that are side chain protected in STEP1, are selected from the group consisting of Arg, Asn, Asp, Cys, Glu, Gln, His, Lys, Ser, Thr, Trp and Tyr.

The side chain protection of the side chains of the amino acid residues of FRAG 1 and FRAG2 is preferably realized in form of side chain protection groups which are not cleavable under weakly acidic conditions, with the weakly acidic conditions as described herein.

Preferably, side chain protection of Cys is Trt or Acm.

Preferably, side chain protection of Asn and Gln is Trt.

Preferably, side chain protection of His is Trt or Dnp.

Preferably, side chain protection of Glu and Asp is OtBu.

Preferably, side chain protection of Thr, Ser and Tyr is tBu.

Preferably, side chain protection of Trp is Boc.

Preferably, side chain protection of Arg is Pbf, Pmc, Mtr or MIS.

Preferably, side chain protection of Lys is Alloc, Z or Boc.

More preferably, side chain protection of Cys, Asn, His and Gln is Trt;
side chain protection of Glu and Asp is OtBu;
side chain protection of Thr, Ser and Tyr is tBu;
side chain protection of Trp is Boc;
side chain protection of Arg is Pbf;
side chain protection of Lys is Alloc.

Val and Ser, when they occur in the sequence of FRAG1 or of FRAG2 on adjacent positions as Val⁽ⁿ¹⁾ and Ser⁽ⁿ¹⁺¹⁾ or as Val⁽ⁿ²⁾ and Ser⁽ⁿ²⁺¹⁾ respectively, can also be used in the protected form of a pseudoproline Val-Ser[Psi^{(Me,Me)}Pro]-OH.

Also other possible pseudoprolines can be used, depending on the sequence of FRAG1 and FRAG2.

Cleavage of any PROTGN and of psWang, and the cleavage of any side chain protecting group, also the cleavage of the tBu protecting group on the alpha COOH of the Glu of the Palmitoyl-Glu-OtBu residue, can be done separately or simultaneously. The cleavage is preferably done in STEP2. When the cleavage is done simultaneously, then the cleavage is usually called global deprotection.

The conditions of such cleavage reactions are known to the skilled person and are dependent on the nature of protecting group. Many protecting groups require acidic conditions for cleavage, preferably strongly acidic conditions, with the strongly acidic conditions as defined herein, other protecting groups such as Z require cleavage by catalytic hydrogenation.

Strongly acidic conditions means, in the context if this invention, using for cleavage a mixture of from 50 to 100%, preferably from 60 to 100%, more preferably from 70 to 100%, even more preferably from 80 to 100%, of TFA with a component COMPSTRONG, the % are vol-% and are being based on the total volume of the mixture of TFA and COMPSTRONG.

Therefore, TFA can also be used neat.

Preferably, COMPSTRONG is selected from the group consisting of SOLVSTRONG, phenol, water, TIS and a mixture thereof;

SOLVSTRONG is solvent that is inert against TFA.

Preferably, SOLVSTRONG is DCM. more preferably, COMPSTRONG is a mixture of phenol and TIS or a mixture of phenol, water and TIS.

Preferably, the volume ratios of a mixture TFA : water : TIS are
TFA: from 80 to 98;
water: from 1 to 10;
TIS: from 1 to 10;
with the individual volume ratios of the three components adding up to 100.

Preferably, the volume ratios of a mixture TFA : phenol: water : TIS are
TFA: from 70 to 97;
phenol: from 1 to 10;
water: from 1 to 10;
TIS: from 1 to 10;
with the individual volume ratios of the four components adding up to 100.

Preferably, the total amount of the mixture is from 5 to 20 ml per g of protected peptide.

Preferably, the cleavage is done at atmospheric pressure.

Preferably, the reaction temperature of the cleavage is from -20 to 70°C; more preferably from -15 and 40°C, even more preferably from -15 to 35°C.

Preferably, the reaction time of the cleavage is from 30 min to 12 h, more preferably from 30 min to 6 h, even more preferably from 30 min to 4 h.

Any SPPS can be done on any conventional resin.

Preferably, the resin for the SPPS of FRAG2 is a resin that allows to connect the psWang linker covalently to the resin with an ESTBOND2;
more preferably, with an ESTBOND2 that is cleavable under weakly acidic conditions, with the weakly acidic conditions as defined herein.

Preferably, FRAG1 is prepared by SPPS.

Preferably, the C-terminal amino acid of FRAG1 is connected to the resin used for the SPPS with an ester bond ESTBOND1;
so the resin used for the SPPS of FRAG1 is a resin that allows to connect the C-terminal amino acid of FRAG1 to the resin with ESTBOND1.

Preferably, ESTBOND1 is cleavable under weakly acidic conditions, with the weakly acidic conditions as defined herein.

Preferably, the alpha COOH of the C-terminal AA of FRAG1 is covalently connected to the resin used for the SPPS.

Resins used for SPPS, that allow to connect the psWang linker or the C-terminal amino acid of FRAG1 covalently to the resin with an ESTBOND1 or with an ESTBOND2 respectively that are cleavable under weakly acidic conditions, with the weakly acidic conditions as defined herein, are for example CTC resin, Rink acid resin, SASRIN resin, resin modified with HAL and resin modified with HMPB.

So preferably, SPPS is done on a resin selected from the group consisting of CTC resin, Rink acid resin, SASRIN resin, resin modified with HAL and resin modified with HMPB.

Preferably, SPPS is done on a CTC resin.

More preferably, FRAG1 and FRAG2 are prepared by SPPS on a CTC resin.

Even more preferably, FRAG2 is prepared by SPPS on a CTC resin that has been loaded with the psWang linker prior to the coupling of the C-terminal AA to the CTC resin.

Preferably, any SPPS is done by stepwise coupling of the individual amino acids.

Preferably, FRAG1 and FRAG2 are prepared by SPPS using Fmoc/tBu strategy; with Fmoc as protecting group for the alpha NH₂ of the amino acids used in the SPPS;
more preferably except for the N-terminal amino acid of FRAG1, where the alpha NH₂ is protected with PROTGN.

Weakly acidic conditions means, in the context if this invention, using for cleavage a mixture of from 0.01 to 25%, preferably from 0.01 to 15%, more preferably from 0.05 to 10%, even more preferably from 0.1 to 7.5%, TFA in a solvent SOLVWEAK, the % are vol-% and are being based on the total volume of the mixture of TFA and SOLVWEAK.

SOLVWEAK is any solvent which is inert against TFA.

Preferably SOLVWEAK is DCM, methyl THF or a mixture thereof.

Preferably, the total amount of the mixture is from 5 to 20 ml per g of protected peptide. Preferably, the cleavage is done at atmospheric pressure.

Preferably, the reaction temperature of the cleavage is from -20 to 70°C; more preferably from -15 and 40°C, even more preferably from -15 to 35°C.

Preferably, the reaction time of the cleavage is from 5 min to 12 h, more preferably from 5 min to 6 h, even more preferably from 10 min to 4 h.

Cleavage under weakly acidic conditions can also be done using HFIP instead of TFA.

Preferably, ESTBOND1 and ESTBOND2 are cleaved under weakly acidic conditions.

In one embodiment, PEP is liraglutide, and the (*) in formula (psWang) denotes the covalent bond to the C atom of the CO residue of Gly⁽³¹⁾ of liraglutide; also with all the embodiments described herein.

Preferably,
FRAG1 is Boc-H⁽¹⁾AEGT⁽⁵⁾FTSDV⁽¹⁰⁾SSYLE⁽¹⁰⁾G⁽¹⁶⁾-OH; and
FRAG2 is H-Q⁽¹⁷⁾AAK⁽²⁰⁾(Palmitoyl-Glu-OtBu)EFIAW⁽²⁵⁾LVRGR⁽³⁰⁾G⁽³¹⁾-psWang.

FRAG1 can also be depicted as Boc-[1-16]-OH or as compound of formula (I). 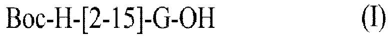
FRAG2 can also be depicted as H-[17-31]-psWang or as compound of formula (II).

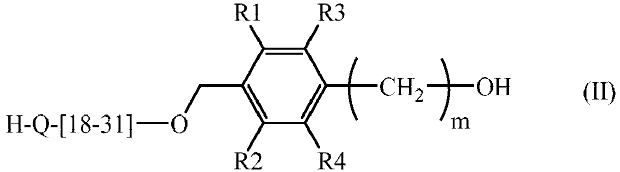

Liraglutide with psWang protection on the C-terminal COOH can also be depicted as compound of formula (III).

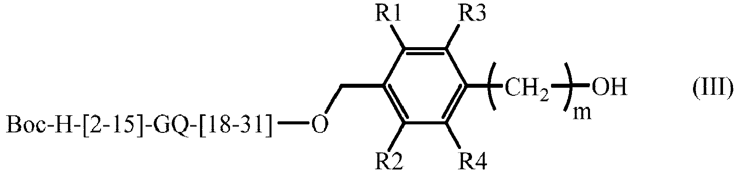

Preferably, Val⁽¹⁰⁾ and Ser⁽¹¹⁾ are present in FRAG1 of liraglutide in form of a pseudoproline Val⁽¹⁰⁾-Ser⁽¹¹⁾(psi^{Me,Me}Pro).

More preferably, FRAG1 has the sequence Boc-His⁽¹⁾(Trt)-Ala-Glu(OtBu)-Gly-Thr⁽⁵⁾(tBu)-Phe-Thr(tBu)-Ser(tBu)-Asp(OtBu)-[Val⁽¹⁰⁾-Ser(psi^{Me,Me}Pro)]-Ser(tBu)-Tyr(tBu)-Leu-Glu⁽¹⁵⁾(OtBu)-Gly⁽¹⁶⁾-OH, and is also called protected fragment 1.

More preferably, FRAG2 has the sequence H-Gln⁽¹⁷⁾(Trt)-Ala-Ala-Lys⁽²⁰⁾(Palmitoyl-Glu-OtBu)-Glu(OtBu)-Phe-Ile-Ala-Trp⁽²⁵⁾(Boc)-Leu-Val-Arg(Pbf)-Gly-Arg⁽³⁰⁾(Pbf)-Gly-psWang, and is also called protected fragment 2.

In case of liraglutide,
preferably, Val⁽¹⁰⁾ and Ser⁽¹¹⁾ are used in the SPPS of FRAG1 in form of pseudoproline Fmoc-Val-Ser[Psi^{(Me,Me)}Pro]-OH.

The Palmitoyl-Glu-OtBu residue on the N⁶ of the Lys⁽²⁰⁾ of FRAG2 is the residue of formula (PALGLU), 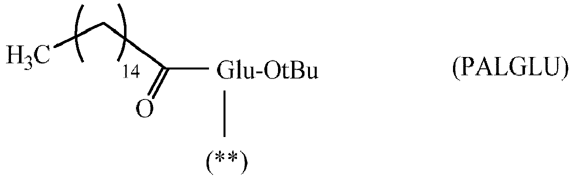
with the (**) in formula (PALGLU) denoting the covalent bond between the COOH of the side chain of the Glu and the N⁶ of the Lys⁽²⁰⁾,
and the Palmitoyl-Glu-OtBu residue is covalently bonded to the Lys⁽²⁰⁾ in a reaction REACPALGLU, wherein the NH₂ of the side chain of the Lys is reacted with a precursor of the residue of formula (PALGLU);
REACPALGLU is done before FRAG2 is cleaved from the resin.

So REACPALGLU is done on-resin, that is REACPALGLU is done while FRAG2 or a precursor of FRAG2 is still covalently bonded to the resin.

Preferably, the precursor of the residue of formula (PALGLU) is Pal-Glu(OSu)-OtBu, that is compound of formula (10); 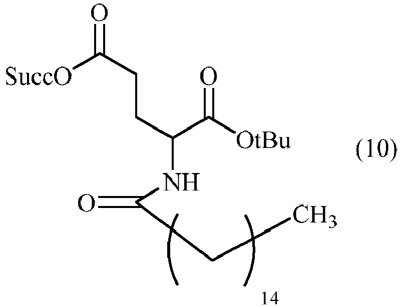 with SuccO being residue of formula (SuccO); 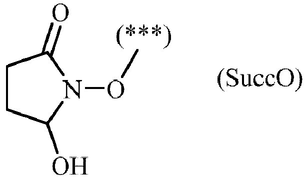 wherein the (***) denotes the covalent bond to the CO residue in compound of formula (10).

Preferably, REACPALGLU is done after the coupling of Ala⁽¹⁹⁾ or after the coupling of Ala⁽¹⁸⁾, and before removal of the protecting group of the alpha NH₂ of Ala⁽¹⁹⁾ or of Ala⁽¹⁸⁾ respectively, preferably after the coupling of Ala⁽¹⁸⁾.

Preferably, any protecting group of the side chain of Lys⁽²⁰⁾ is cleaved before REACPALGLU.

In case of liraglutide,
preferably, Lys⁽²⁰⁾ is used in the SPPS of FRAG2 in form of Fmoc-Lys(Alloc)-OH;
so the side chain protecting group of Lys⁽²⁰⁾, which is cleaved before REACPALGLU, is preferably Alloc.

Preferably, cleavage of Alloc from the Lys⁽²⁰⁾ is done with a reaction CLEAVALLOC using Pd(PPh₃)₄ and PhSiH.

CLEAVALLOC can be done in the presence of an additive ADDALLOC, ADDALLOC is selected from the group consisting of morpholine, 1,3-dimethylbarbituric acid, pyrrolidine, triphenylphosphine, dimedone, and mixtures thereof.

Preferably, the molar amount of ADDALLOC is from 2 to 7 times of the molar amount of the Alloc residue.

Preferably, the molar amount of Pd(PPh₃)₄ is from 0.01 to 10 times, more preferably from 0.02 to 5 times, even more preferably from 0.05 to 2 times, of the molar loading capacity of the resin.

Preferably, the molar amount of PhSiH is from 1 to 30 times, more preferably from 2 to 20 times, even more preferably from 5 to 15 times, of the molar loading capacity of the resin.

Preferably, CLEAVALLOC is done in a solvent selected from the group consisting of DMSO, dichloromethane, Me-THF, DMF, NMP, and mixtures thereof, more preferably the solvent is DCM.

Preferably, CLEAVALLOC is done at atmospheric pressure.

Preferably, the reaction temperature of CLEAVALLOC is from -20 to 70°C; more preferably from -15 and 60°C, even more preferably from 5 to 35°C.

Preferably, the reaction time of CLEAVALLOC is from 1 min to 2 h.

Preferably, any coupling is for the ease of reading also called COUPL, such as a SPPS coupling reaction or such as the LPPS coupling reaction of FRAG1 and FRAG2, and can be carried out using reaction conditions known in the art of peptide synthesis.

Preferably, coupling reagents, which can be used for COUPL and which are used in situ, are for example phoshonium or uronium coupling reagents, like benzotriazol-1-yloxy-tris(dimethylamino)phosphonium hexafluorophosphate (BOP), benzotriazol-1-yloxy-tris(pyrrolidino )phosphonium hexafluorophosphate (PyBOP), O-(benzotriazol-1-yl)-1,1,3,3-tetramethyluronium hexafluorophosphate (HBTU), O-(6-chlorobenzotriazol-1-yl)-1,1,3,3-tetramethyluronium hexafluorophosphate (HCTU), O-(6-chlorobenzotriazol-1-yl)-1,1,3,3-tetramethyluronium tetrafluoroborate (TCTU), O-(7-azabenzotriazol-1-yl)-1,1,3,3-tetramethyluronium hexafluorophosphate (HATU), O-(7-azabenzotriazol-1-yl)-1,1,3,3-tetramethyluronium tetrafluoroborate (TATU), O-(benzotriazol-1-yl)-1,1,3,3-tetramethyluronium tetrafluoroborate (TBTU), O-[cyano(ethoxycarbonyl)methylenamino]-1,1,3,3-tetramethyluronium tetrafluoroborate (TOTU) and (1-cyano-2-ethoxy-2-oxoethylidenaminooxy)dimethylamino-morpholino-carbenium hexafluorophosphate (COMU);
or carbodiimide coupling reagents, like diisopropylcarbodiimide (DIC), dicyclohexylcarbodiimide (DCC) and water-soluble carbodiimides (WSCDI) like 1-ethyl-3-(3-dimethylaminopropyl)carbodiimide (EDC), optionally as salt like as hydrochloride salt.

Other coupling techniques use pre-formed active esters, such as N-hydroxysuccinimide (HOSu) and p-nitrophenol (HONp) esters, pre-formed symmetrical anhydrides, non-symmetrical anhydrides such as N-carboxyanhydrides (NCAs) and acid halides, such as acyl fluorides or acyl chlorides.

Preferred coupling reagents are carbodiimide coupling reagents and phoshonium coupling reagents, most preferred coupling reagents are selected from group consisting of PyBOP, DCC, DIC and EDC;
more preferably, coupling reagent is PyBOP or DIC.

EDC is preferably used as a salt, more preferably as EDC.HC1.

Preferably, the molar amount of coupling reagent is from 1 to 10 times, more preferably from 1 to 5 times, of the molar loading capacity of the resin.

COUPL can be done in the presence of a base, preferably a tertiary amine base, which both deprotonates the COOH residue of the carboxy component and neutralizes any counterion of the NH₂ residue of the amino component in COUPL, and thus facilitates the coupling reaction.

Suitable bases are for example trialkylamines, like N,N-diisopropylethylamine (DIPEA) or triethylamine (TEA); N,N-dialkylanilines, like N,N-diethylaniline; 2,4,6-trialkylpyridines, like 2,4,6-trimethylpyridine; N,N-dialkylaminopyridines, like N,N-4-dimethylaminopyridine; and N-alkylmorpholines, like N-methylmorpholine.

Preferably, COUPL is done in the presence of DIPEA as a base.

Preferably, the molar amount of base is from 0.01 to 20 times, more preferably from 0.02 to 10 times, of the molar loading capacity of the resin.

COUPL can be done in the presence of an auxiliary nucleophile as additive due to their positive effect in suppressing undesired side reactions. Any known auxiliary nucleophile may be used.

Examples of suitable auxiliary nucleophiles are ethyl (hydroxyimino)cyanoacetate, 1-hydroxybenzotriazole (HOBt), N-hydroxysuccinimide (HOSu), N-hydroxy-3,4-dihydro-4-oxo-1,2,3-benzotriazine (HOOBt) and 1-hydroxy-7-azabenzotriazole (HOAt).

Preferably, HOBt or HOAt are used as auxiliary nucleophile.

Preferably, the molar amount of auxiliary nucleophile is from 0.5 to 3 times of the molar amount of the amino residue that is to be coupled.

Especially, COUPL is done using DIC/OxymaPure, DCC/HOBt, EDC/HOBt, PyBOP/HOBt or EDC/HOAt.

Preferably, COUPL can be done in any inert solvent which can dissolve the reactants. Preferred solvents for COUPL are water-miscible solvents like dimethyl sulfoxide (DMSO), dioxane, tetrahydrofurans such as tetrahydrofurane (THF) or methyltetrahydrofurane (Methyl-THF), 1-methyl-2-pyrrolidone (NMP), N,N-Dimethylformamide (DMF), N,N-Dimethylacetamide (DMA), or any mixture thereof; and non water-miscible solvents like dichloromethane (DCM), ethyl acetate or any mixture thereof; and any suitable mixture between water-miscible and non water-miscible solvents, including mixtures with water.

More preferably, COUPL is done in a solvent selected from the group consisting of DMSO, dichloromethane, Me-THF, DMF, NMP, and mixtures thereof.

Preferably, COUPL is done at atmospheric pressure.

Preferably, the reaction temperature of COUPL is from -20 to 70°C; more preferably from -15 and 40°C, even more preferably from -15 to 35°C.

Preferably, the reaction time of COUPL is from 30 min to 48 h, more preferably from 30 min to 24 h, even more preferably from 30 min to 12 h, especially from 30 min to 6 h.

Preferably, the progress of COUPL is monitored by HPLC and thereby the necessary reaction time is determined.

Preferably, the coupling of the first AA onto the resin in case of FRAG1 is an esterification reaction ESTERREACAA1.

ESTERREACAA1 provides for ESTBOND1.

The coupling of the first AA onto the psWang linker connected to a resin in case of FRAG2 is an esterification reaction ESTERREACAA2.

Both ESTERREACAA1 and ESTERREACAA2 can be done with the reagents and the conditions as described for COUPL.

ESTERREACAA1 can be done without using a coupling reagent.

Preferably, the molar amount of Pal-Glu(OSu)-OtBu in REACPALGLU is from 1 to 15 times, more preferably from 1 to 10 times, even more preferably from 1 to 5 times, of the molar loading capacity of the resin.

REACPALGLU can be done in the presence of a base, preferably a tertiary amine base. Suitable bases are for example trialkylamines, like N,N-diisopropylethylamine (DIPEA) or triethylamine (TEA); N,N-dialkylanilines, like N,N-diethylaniline; 2,4,6-trialkylpyridines, like 2,4,6-trimethylpyridine; and N-alkylmorpholines, like N-methylmorpholine.

Preferably, REACPALGLU is done in the presence of DIPEA as a base.

Preferably, the molar amount of base is from 1 to 10 times, more preferably from 3 to 8 times, of the molar loading capacity of the resin.

Preferably, REACPALGLU is done in a solvent selected from the group consisting of DMSO, dichloromethane, Me-THF, DMF, NMP, and mixtures thereof, more preferably the solvent is DCM.

Preferably, REACPALGLU is done at atmospheric pressure.

Preferably, the reaction temperature of REACPALGLU is from -20 to 70°C; more preferably from -15 and 60°C, even more preferably from 5 to 35°C.

Preferably, the reaction time of REACPALGLU is from 1 to 48 h, more preferably from 5 h to 24 h, even more preferably from 10 h to 24 h.

Fmoc cleavage is known to the skilled person, it is done with a base, preferably the base is a secondary amine, more preferably piperidine.

The base is used in excess with regard to the molar loading capacity of the resin.

Preferably, DMF is used as solvent in the Fmoc cleavage reaction.

Preferably, the Fmoc cleavage is done at atmospheric pressure.

Preferably, the reaction temperature of the Fmoc cleavage is from -20 to 70°C; more preferably from -15 and 40°C, even more preferably from -15 to 35°C.

Preferably, the reaction time of the Fmoc cleavage is from 1 min to 12 h, more preferably from 1 min to 6 h, even more preferably from 1 min to 1 h.

PEP can be isolated, preferably after a global deprotection, according to standard methods known to the skilled such as precipitation, preferably with ether, centrifugation, filtration etc.

PEP can be purified according to standard methods known to the skilled person, such as HPLC.

### Examples

### AA

The SPPS was done with the following AA, if not stated otherwise:
Boc-His(Trt)-OH
Fmoc-Ala-OH
Fmoc-Glu(OtBu)-OH
Fmoc-Gly-OH
Fmoc-Thr(tBu)-OH
Fmoc-Phe-OH
Fmoc-Ser(tBu)-OH
Fmoc-Asp(OtBu)-OH
Fmoc-Tyr(tBu)-OH
Fmoc-Val-OH
Fmoc-Val-Ser[Psi^{(Me,Me)}Pro]-OH for position (10) and (11);
Fmoc-Leu-OH
Fmoc-Gln(Trt)-OH
Fmoc-Lys(Alloc)-OH
Fmoc-Ile-OH
Fmoc-Trp(Boc)-OH
Fmoc-Arg(Pbf)-OH

**Pal-Glu(OSu)-OtBu,** that is compound of formula (10), was purchased from IRIS Biotech

### METHODS

### Ninhydrin Test, also called Kaiser Test

The test is known to the skilled person, see Kaiser E. et al., Analytical Biochemistry 1970, 34, 595-598.
Solution 1: Dissolve 5 g ninhydrin in 100 mL ethanol.
Solution 2: Dissolve 40 g phenol in 10 mL ethanol.
Solution 3: Add 2 mL of a 0.001 M aq. KCN solution to 98 mL pyridine.

### Procedure:

1. Wash resin with DCM (2 times).
2. Sample a few beads of resin in an Eppendorf tube.
3. Add one to two drops of each of the three solutions 1 to 3.
4. Mix well and heat to 120 °C for 3 to 5 min.
5. The presences of free resin-bound amines are indicated by blue resin beads.

### Coupling

SPPS was done manually.

### Washing

In the following examples washing with a solvent, such as DMF or DCM, was done by filtering the resin, suspending and stirring the resin in the solvent for a certain time and filtering again.

For washes and Fmoc removal ca. 5 volumes of resin of the solvent was used; for couplings ca. 20 ml/g resin was used, if not stated otherwise.

Stated in the examples are the number of repetitions of this washing cycle together with the time for stirring. Also in case of treating the resin with a reagent that is dissolved in a solvent this description of the number of repetitions of this treatment together with the time for stirring is used in the example, if not otherwise stated.

### Yield

The yield was calculated on the basis of the synthesis scale with respect to resin loading capacity f and resin weight, if not otherwise stated.

### Method for determination of purity

Peptide-resin (100 mg) is treated with ca. 2 ml of TFA : H₂O : TIS in the volume ratio 94:3:3 and stirring for 1 h at RT. The suspension is filtered. The deprotected peptide is precipitated by addition of diisopropyl ether (20 ml) to the filtrate. The purity of crude deprotected fragment is checked by HPLC:
(A) Determination of purity of fragment 1:
   - Column with C18 stationary phase
   - Mobile phase A: 0.1% (v/v) TFA in water
   - Mobile phase B: 0.1% (v/v) TFA in acetonitrile
   - Gradient: 5% (v/v) B to 100% (v/v) B in 11 min
   - Temperature: RT
   - Flow rate : 1 ml/min
   - Detection: 254 nm
(B) Determination of purity of fragment 2:
   - Column with C18 stationary phase
   - Mobile phase A: 0.1% (v/v) TFA in water
   - Mobile phase B: 0.1% (v/v) TFA in acetonitrile
   - Gradient: 5% (v/v) B to 100% (v/v) B in 13 min
   - Temperature:40°C
   - Flow rate : 1 ml/min
   - Detection: 254 nm
(C) Determination of purity of crude liraglutide:
   - Column with C18 stationary phase
   - Mobile phase A: 0.1% (v/v) TFA in water
   - Mobile phase B: 0.1% (v/v) TFA in acetonitrile
   - Gradient: 5% (v/v) B to 100% (v/v) B in 11 min
   - Temperature: RT
   - Flow rate : 1 ml/min
   - Detection: 254 nm

### Fmoc determination, also used for determination of resin loading:

An analytical method based on the UV absorption of dibenzofulvene after cleavage of Fmoc on an aliquot of peptide-resin, the method is known to the skilled person. It can be done as follows:
An aliquot of loaded resin (ca. 100 mg) is washed with DMF (3 times with 5 ml) and DCM (3 times with 5 ml). It is then dried under vacuum.

10 mg of dried resin is added to a 2 mL polypropylene tube, 0.8 mL of DMF is added and the resin is allowed to swell for 10 minutes. Then 0.2 mL of piperidine is added, then the mixture is shaken for 20 min. Then 10 microL of the supernatant is pipetted into a quartz cuvette and is diluted with 0.990 mL of DMF. The absorbance at lamda = 301 nm is determined. The piperidine-dibenzofulvene adduct that is formed upon Fmoc deprotection has a molar extinction coefficient of epsilon = 7800 L x mol⁻¹ x cm⁻¹; thus, the loading value is determined using Beer-Lambert law.

### Apparent pH

The determination of the apparent pH is a way to measure the pH in an organic solvent (that is in an non aqueous solvent). The procedure is well known to the skilled person: One drop of the organic solvent or of the organic solvent solution is put on a wet pH paper stick, the resulting coloration gives the apparant pH.

### Example 1: Synthesis of Liraglutide using a pseudo Wang linker

Synthesis of Liraglutide using a pseudo Wang linker was done according to Scheme 1:

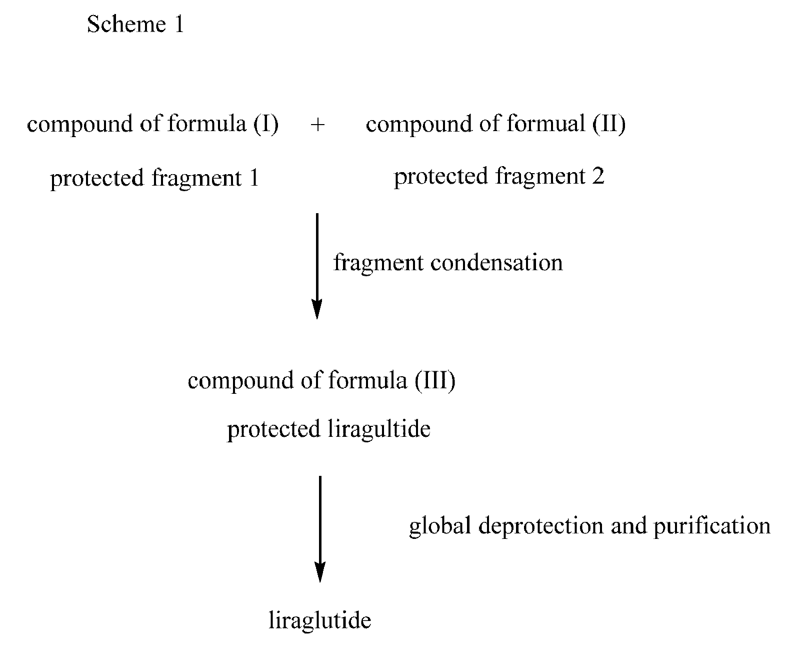

### Example 1-1: SPPS of protected fragment 1 with pseudoproline

Protected fragment 1, that is protected Boc-His-(2-15)-Gly-OH, was synthesized by conventional Fmoc SPPS according to Scheme 2, wherein RESIN1 was a CTC resin, purchased from IRIS BioTech with loading capacity f= 1.6 mmol/g, Val⁽¹⁰⁾ and Ser⁽¹¹⁾ were coupled in form of a pseudo-Pro, Fmoc-Val-Ser(psi^{Me,Me}pro)-OH, the His⁽¹⁾ was coupled as Boc-His⁽¹⁾(Trt)-OH.

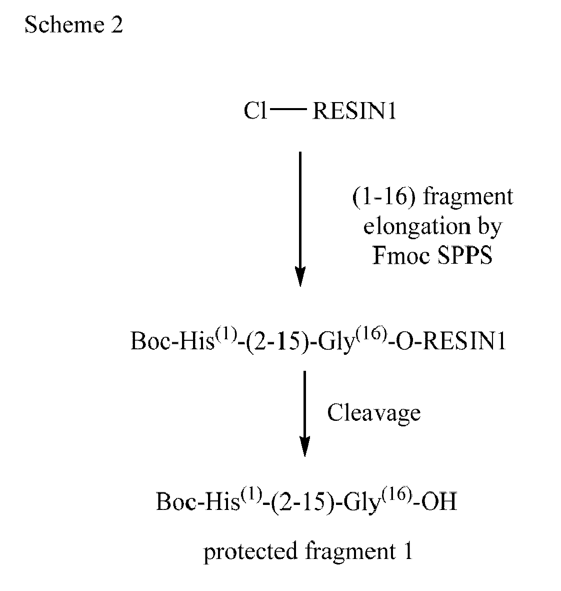

The resin (2.01 g) was washed with DCM (3 times with 20 ml). Then the loading capacity of the resin was reduced to f = 0.8 mmol/g by the addition of Fmoc-Gly-OH (0.5 eq; 0.478 g) and DIPEA (2 eq) in DCM (40 ml) and subsequent stirring for 2 h. Then the loading was checked by Fmoc determination and it was found to be f = 0.71 mmol/g.

Stepwise coupling of the other AAs was carried out at RT by adding the reagents AA : DIC : OxymaPure in the ratio 3 eq : 3 eq : 3 eq in 40 ml DMF and then stirring for 90 min. Removal of the Fmoc was done by treatment at RT with piperidine : DMF (2:8 v/v; 5 ml/g resin) for 1 time for 1 min and 1 time for 15 min. After each coupling, a ninhydrin test was done. In all cases except for Phe⁽⁶⁾ and His⁽¹⁾, the ninhydrin test was negative. For Phe⁽⁶⁾ and His⁽¹⁾, the coupling was repeated with stirring for 120 min instead of 90 min, and then the ninhydrin test was negative.

### Cleavage of the peptide - resin bond

After the introduction of the last amino acid, the resin was washed with DCM (5 ml/g; 5 time for 1 min), then washed with MeOH (5 ml/g; 5 time for 1 min) and dried. The protected fragment 1 was then cleaved from the resin using TFA/DCM (2/98 v/v; 40 ml; 3 times for 5 min). The TFA/DCM filtrates were collected and evaporated under vacuum. The residue was precipitated by addition of diisopropyl ether (40 ml), and isolated by filtration and drying under vacuum.

Protected fragment 1 (2.93 g) was isolated with 81 % yield and 92% purity.

The purity was determined by HPLC.

### Example 1-3: SPPS of protected fragment 2

Protected fragment 2, that is protected H-Gln-(18-31)-psWang, was synthesized by conventional Fmoc SPPS according to scheme 3, wherein RESIN2 was a CTC resin purchased from IRIS Biotech.

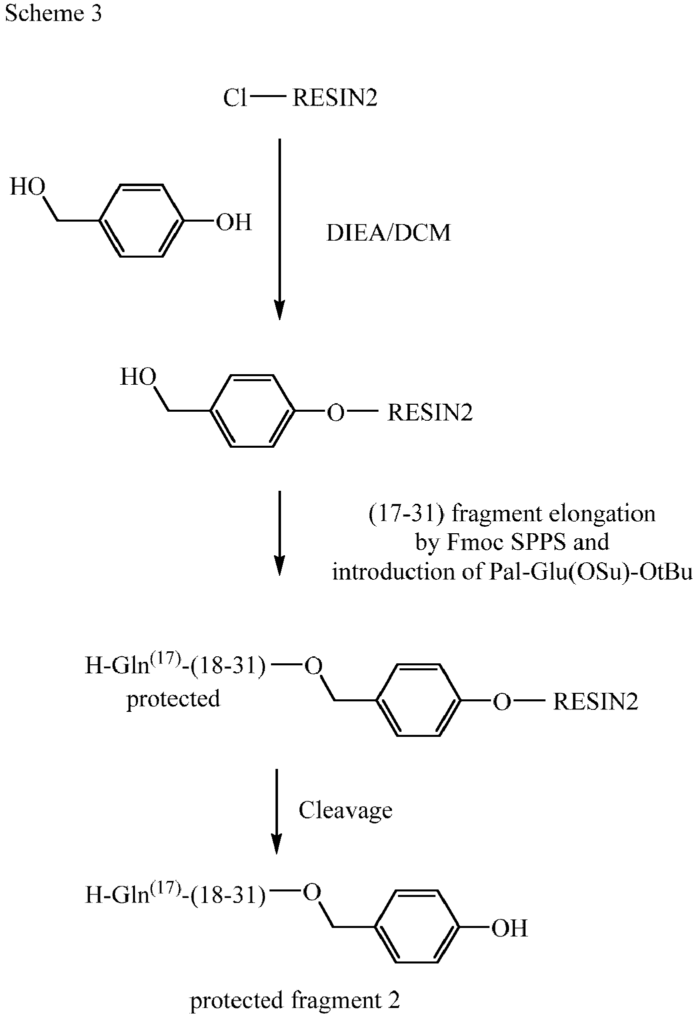

4-Hydroxymethylphenol (0.3 eq) and DIPEA (2 eq) in DMF (60 ml) were added to the 2-CTC-resin (3 g, f = 1.6 mmol/g), and after stirring for 2 h, MeOH (10 ml) was added to cap the unreacted Cl. Then Fmoc-Gly-OH (3 eq taking into account a loading of 0.5 mmol/g) was added together with DIC (3 eq) and DMAP (0.3 eq) in DMF (60 ml) and stirred for 90 min. Then the resin was treated for 10 min with Ac₂O : DIPEA 0.141 ml : 0.26 ml (1 eq) and the final loading as calculated by Fmoc determination was 0.45 mmol/g; the Fmoc determination also served for cleavage of Fmoc.

Coupling of the remaining AA was done by treatment at RT with AA : DIC : OxymaPure (3 eq:3 eq :3 eq) for 90 min. Removal of the Fmoc after each coupling was done by treatment at RT with piperidine : DMF (2:8 v/v, 5 ml/g resin) for 1 time for 1 min and 1 time for 15 min. After each coupling, the ninhydrin test was carried out. In all cases except Phe⁽²²⁾, Ala¹⁸ and Gln⁽¹⁷⁾(Trt), the ninhydrin test was negative. For Phe⁽²²⁾, Ala⁽¹⁸⁾, and Gln⁽¹⁷⁾(Trt), the coupling was repeated with stirring for 120 min instead of 90 min and then the ninhydrin test was negative.

### Incorporation of Pal-Glu(OSu)-OtBu

After the coupling of Ala⁽¹⁸⁾ and before the removal of its Fmoc protecting group, the Alloc protecting group of the side chain of Lys⁽²⁰⁾ was removed [by (i) washing the resin with DCM (5 times for 1 min each); (ii) treatment of the resin at RT with Pd(PPh₃)₄ : PhSiH in the ratio 0.1eq : 10 eq (2 times for 10 min each in DCM); (iii) washing with DCM (5 times for 1min each)], thereafter the resin was treated at RT with Pal-Glu(OSu)-OtBu (3 eq) and DIPEA (6 eq) in DCM (20 ml/g resin) for 16 hours. The ninhydrin test confirmed that the reaction was finished and the coupling of the last amino acid Fmoc-Gln(Trt)-OH was done .followed by the N-terminal Fmoc deprotection. In order to completely remove the traces of piperidine, the peptide resin is extensively washed with DMF (5 times 50 ml DMF for 1 min each), DCM (5 times 50 ml DMF for 1 min each), 0.01% TFA in DCM (5 times 50 ml DMF for 1 min each).

### Cleavage of peptide-resin bond

The peptide -resin bond is cleaved by means of 2% v/v TFA in DCM (3 times 50 ml for 5 min).

The filtrates are collected and evaporated under vacuum. The residue is precipitated in water (100 ml), filtered and dried under vacuum at 30±5°C.

### Yield was ca 50%, purity was ca 85%

The purity was determined by HPLC.

### Example 1-4: Coupling of protected Fragments 1 and 2 to provide protected liraglutide

Protected fragment 2 (900.0 mg, 1.0 eq, prepared according to example 1-3) was dissolved in DMSO (25 mL), the resulting solution was added to a solution of protected fragment 1 (795.5 mg, 1.1 eq), prepared according to example 1-1, in DMSO (25 mL), then PyBOP (1.1 eq) was added and the apparent pH is adjusted to 7.5 to 8.5 by dropwise addition of DIPEA. After 2.5 h the reaction was quenched by precipitation into 5 wt% aqueous NaHCO₃. The solid obtained after centrifugation was washed with 5 wt% aqueous citric acid. Yield: ca. 100% (ca. 1.6 g as it is).

### Example 1-5: Global deprotection of protected liraglutide:

The protected liraglutide (1.8 g, prepared according to example 1-4) was added to ca. 18 ml of a mixture of TFA : phenol: water : TIS (88 : 5 : 5 : 2 v/v) of 0°C. The mixture was stirred 2 h at 0°C and then poured into ether (200 ml) of 0°C. The precipitate was isolated by centrifugation and was washed with ether (3 times with 20 ml) and was dried under vacuum at 20±5 °C.

Yield ca. 100% (1.3 g as it is)

Purity: 71%

### Example 1-6: Purification of liraglutide

The following purification method is standard purification method which is not optimized for liraglutide and yield.

Liraglutide, prepared according to example 1-5, was purified on XSelect® CSH C18 semi-preparative HPLC column (Waters).
Mobile phase A: 0.1% (v/v) TFA in water
Mobile phase B: 0.1% TFA (v/v) in acetonitrile

**Gradient:**

| **t** | **A** | **B** |
|---|---|---|
| **[min]** | **[%]** | **[%]** |
| **0** | 95 | 5 |
| **3** | 95 | 5 |
| **4** | 51 | 49 |
| **9** | 48.5 | 51.5 |
| **10** | 0 | 100 |
| **12** | 0 | 100 |
| **12.5** | 95 | 5 |

Liraglutide elutes at about minute 7 of the gradient.
Purity: >99%
Purification yield: 10%

## Claims

1. Method for the preparation of a peptide PEP which comprises a coupling of two peptide fragments, an N-terminal fragment FRAG1 of PEP and a C-terminal fragment FRAG2 of PEP, by liquid phase peptide synthesis;
wherein the C-terminal COOH of FRAG2 is protected by a psWang residue;
the psWang residue is residue of formula (psWang); 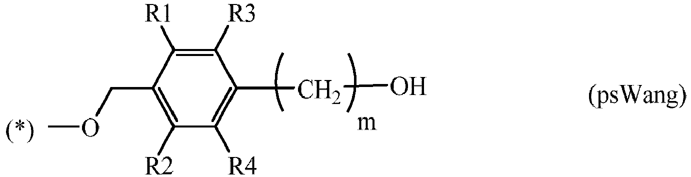
wherein the (*) denotes the covalent bond to the C atom of the CO residue of the C-terminal COOH of FRAG2;
R1, R2, R3 and R4 are identical or different and independently from each other selected from the group consisting of H, C₁₋₄ alkyl, C₁₋₄ alkoxy, CN, F, Cl and Br;
m is 0, 1, 2, 3, 4, 5, 6, 7, 8, 9 or 10.

2. Method according to claim 1, wherein
R1, R2, R3 and R4 are identical or different and independently from each other selected from the group consisting of H, C₁₋₂ alkyl, C₁₋₂ alkoxy and Cl.

3. Method according to claim 1 or 2, wherein
m is 0, 1, 2, 3 or 4.

4. Method according to one or more of claims 1 to 3, wherein
m is 0 and R1, R2, R3 and R4 are H.

5. Method according to one or more of claims 1 to 4, wherein
FRAG2 is prepared by SPPS.

6. Method according to claim 5, wherein
the psWang residue is connected to the resin used for the SPPS by an ester bond ESTBOND2.

7. Method according to claim 6, wherein
wherein ESTBOND2 is cleavable under weakly acidic conditions.

8. Method according to one or more of claims 1 to 7, wherein
the method comprises two steps, a step STEP 1 and a step STEP2;
STEP1 comprises the coupling of FRAG1 and FRAG2 by liquid phase peptide synthesis;
STEP1 provides a peptide PEP-psWang;
STEP2 comprises cleavage of psWang from PEP-psWang obtained in STEP1.

9. Method according to one or more of claims 1 to 8, wherein
the N-terminal NH₂ of FRAG1 is protected by a protecting group PROTGN during the coupling of FRAG 1 and FRAG2.

10. Method according to one or more of claims 1 to 9, wherein
any amino acid residue of FRAG 1 and FRAG2, that can be used in a side chain protected form, is used in side chain protected form.

11. Method according to one or more of claims 1 to 10, wherein
FRAG1 is prepared by SPPS.

12. Method according to claim 11, wherein
the C-terminal amino acid of FRAG1 is connected to the resin used for the SPPS with an ester bond ESTBOND1.

13. Method according to claim 12, wherein
ESTBOND1 is cleavable under weakly acidic conditions.

14. Method according to one or more of claims 5 to 13, wherein
SPPS is done on a resin selected from the group consisting of CTC resin, Rink acid resin, SASRIN resin, resin modified with HAL and resin modified with HMPB.

15. Method according to one or more of claims 5 to 14, wherein
SPPS is done on a CTC resin.

16. Method according to one or more of claims 1 to 15, wherein
FRAG1 or FRAG2 are prepared by SPPS using Fmoc/tBu strategy.

17. Method according to one or more of claims 1 to 16, wherein
PEP is liraglutide, and the (*) in formula (psWang) denotes the covalent bond to the C atom of the CO residue of Gly⁽³¹⁾ of liraglutide.

18. Method according to one or more of claims 1 to 17, wherein
FRAG1 is Boc-H⁽¹⁾AEGT⁽⁵⁾FTSDV⁽¹⁰⁾SSYLE⁽¹⁵⁾G⁽¹⁶⁾-OH; and
FRAG2 is H-Q⁽¹⁷⁾AAK⁽²⁰⁾(Palmitoyl-Glu-OtBu)EFIAW⁽²⁵⁾LVRGR⁽³⁰⁾G⁽³¹⁾-psWang.

19. Method according to claim 18, wherein
the Palmitoyl-Glu-OtBu residue on the N⁶ of the Lys⁽²⁰⁾ of FRAG2 is the residue of formula (PALGLU), 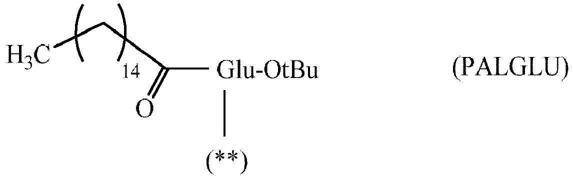
with the (**) in formula (PALGLU) denoting the covalent bond between the COOH of the side chain of the Glu and the N⁶ of the Lys⁽²⁰⁾,
and the Palmitoyl-Glu-OtBu residue is covalently bonded to the Lys⁽²⁰⁾ in a reaction REACPALGLU, wherein the NH₂ of the side chain of the Lys is reacted with a precursor of the residue of formula (PALGLU);
REACPALGLU is done before FRAG2 is cleaved from the resin.

20. Method according to claim 19, wherein
the precursor of the residue of formula (PALGLU) is compound of formula (10); 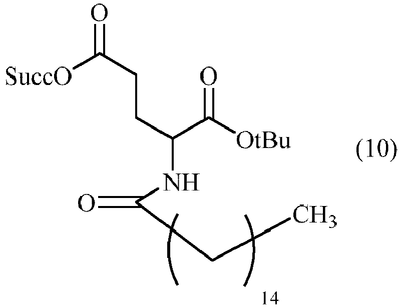 with SuccO being residue of formula (SuccO); 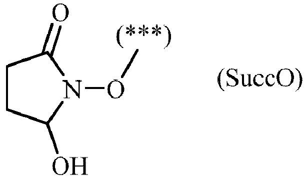 wherein the (***) denotes the covalent bond to the CO residue in compound of formula (10).

21. Method according to claim 19 or 20, wherein
REACPALGLU is done after the coupling of Ala⁽¹⁹⁾ or after the coupling of Ala⁽¹⁸⁾, and before removal of the protecting group of the alpha NH₂ of Ala⁽¹⁹⁾ or of Ala⁽¹⁸⁾ respectively.
